(19) Europäisches Patentamt
European Patent Office
Office européen des brevets

(11) Veröffentlichungsnummer : **0 105 157**
**B1**

(12) # EUROPÄISCHE PATENTSCHRIFT

(45) Veröffentlichungstag der Patentschrift : **21.10.87**

(51) Int. Cl.[4] : **C 07 D307/94**, C 11 B 9/00

(21) Anmeldenummer : **83108155.9**

(22) Anmeldetag : **18.08.83**

(54) **Riechstoffkompositionen mit einem Gehalt an Spirolactonen.**

(30) Priorität : **25.08.82 US 411145**
**16.05.83 US 495212**

(43) Veröffentlichungstag der Anmeldung : **11.04.84 Patentblatt 84/15**

(45) Bekanntmachung des Hinweises auf die Patenterteilung : **21.10.87 Patentblatt 87/43**

(84) Benannte Vertragsstaaten : **CH DE FR GB LI NL**

(56) Entgegenhaltungen :
**DE-A- 2 749 511**
**TETRAHEDRON LETTERS, Nr. 59, Dezember 1970, Seiten 5133-5136, Pergamon Press, Oxford, GB P. PICARD u.a.: "Etude conformationnelle dans la serie de l'oxa-1 spirol (4.5) decane"**
**BULLETIN DE LA SOCIETE CHIMIQUE DE FRANCE, Nr. 3, 1971, Seiten 1075-1080, Paris, FR J. MOULINES et al.: "Composés hétérocycliques spiranniques. I. - Synthèse et détermination de la configuration de quelques oxa-1 spiro (4.5) décanes et décanones-2"**

(73) Patentinhaber : **L. GIVAUDAN & CIE Société Anonyme**
**CH-1214 Vernier-Genève (CH)**

(72) Erfinder : **Dahill, Robert T.**
**8 Sweetbriar Lane**
**Holmdel, N.J. 07733 (US)**
Erfinder : **Golle, Erlinda F.**
**46 Krueger Place**
**Passaic, N.J. 07055 (US)**
Erfinder : **Purzycki, Kenneth L.**
**273 Marcella Road**
**Lake Parsippany, N.J. 07054 (US)**

(74) Vertreter : **Urech, Peter, Dr. et al**
**Grenzacherstrasse 124 Postfach 3255**
**CH-4002 Basel (CH)**

**EP 0 105 157 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Riechstoffkompositionen, die durch einen Gehalt an mindestens einem 8-Alkyl-1-oxaspiro [4.5] decan-2-on der Formel

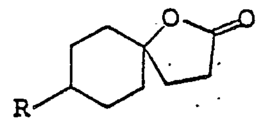

(I)

worin R Methyl, Aethyl, Isopropyl, sek. Butyl und tert. Butyl darstellt, gekennzeichnet sind. Die Formel I soll auch alle möglichen Stereoisomeren von Verbindungen der Formel I umfassen.

Jede der Verbindungen der Formel I ist imstande, einen einzigartigen und höchst individuellen Beitrag in Riechstofformulierungen zu liefern. Es hat sich zwar gezeigt, dass die cis- und die trans-Isomeren der Verbindungen der Formel I in ihren Riechstoffeigenschaften etwas verschieden sind, jedoch komplementieren sich die Gerüche der Isomeren, und als Resultat hat sich herausgestellt, dass vorzugsweise sogar Isomerengemische eingesetzt werden. Die Verbindungen der Formel I haben sich als Komponenten einer ganzen Reihe von Riechstofformulierungen als vorteilhaft erwiesen, beispielsweise in Basen vom holzigen, blumigen, fruchtigen, orientalischen und harzigen Typ.

Die Spirolactone der vorliegenden Erfindung können nach einer Reihe von Methoden dargestellt werden, wobei es sich bei diesen Methoden um in der Literatur beschriebene Verfahren handelt ; siehe diesbezüglich US-PS 2 839 538 ; J. Moulines und R. Lalande, Comptes. Rend. (261), 1983 (1965) ; J. Moulines und R. Lalande, Bull. Soc. Chim. France, 1075-80 (1971) ; G. I. Nikishin und V. D. Vorob'ev, Isvestiya Akademii Nauk SSSR, Otdelenie Khimicheskikh Nauk No. 10 (1962) 1874-1876 ; und C. Walling und E. S. Huyser, Org. Reactions (13), 114 (1963). Das Isopropyl- und das tert.-Butylderivat der Formel I sind bekanntgeworden aus : J. Moulines und R. Lalande, loc. cit., R. M. Jacobson et al. J. Org. Chem. 45, (1980), 349-405.

Was die cis- und trans-Isomeren betrifft, bezieht sich diese Bezeichnungsweise auf diejenigen Verbindungen der Formel I, worin der Alkylrest R und das Sauerstoffatom am Cyclohexylrest entweder cis oder trans zueinander stehen.

Die bevorzugte Methode zur Herstellung der Spirolactone der vorliegenden Erfindung beinhaltet die Umsetzung zwischen Acrylsäure oder einem Ester der Acrylsäure, beispielsweise einem niederen Alkylester, mit vorzugsweise einem Ueberschuss (5-10 Aequivalente) des entsprechenden Cyclohexanols, vorzugsweise in Anwesenheit einer katalytischen Menge eines Initiators für freie Radikale, wie di-t-Butylperoxid (wobei von diesem Initiator ungefähr 0,05 bis 0,5 Aequivalente verwendet werden), und zwar bei einer Temperatur, die genügt, um die Reaktion zu initiieren. Im vorliegenden Fall est ein Temperaturbereich von ungefähr 145-155 °C bevorzugt, wobei sich diese Bevorzugung insbesondere auf den Fall des di-t-Butylperoxides bezieht. Die beschriebene Methode liefert ein Isomerengemisch, das dem Isomerengemisch der Ausgangsalkohole entspricht. Die cis- und trans-Isomeren verfügen über etwas verschiedene Gerüche ; sie können mittels bekannter Techniken, beispielsweise durch Kolonnenchromatographie, Gaschromatographie etc. voneinander getrennt werden ; ökonomischer ist es für die Zwecke der vorliegenden Erfindung allerdings, das Isomerengemisch einzusetzen. Wie oben gesagt, sind die Gerüche der Isomeren komplementär, und schon aus diesem Grund ist das Isomerengemisch gegenüber den individuellen Komponenten in den meisten Fällen bevorzugt.

Die folgende Tabelle I beschreibt die Gerüche der einzelnen Spirolactone der Formel I.

Tabelle I

| R | Geruch |
|---|---|
| $CH_3$ * | stark, sahnig, laktonartig, nach Coumarin, leicht cresylartig |
| $C_2H_5$ * | intensiv, sahnig, laktonartig, leicht holzig |
| iso-$C_3H_7$ | holzig, milchig-laktonartig, trocken-pulverig, |
| sek-$C_4H_9$ * | nach Pfirsich, Aprikose, laktonartig |
| tert-$C_4H_9$ | holzig, Ambra, schwach |

* Neue Verbindungen, die per se wie auch bezüglich ihrer Herstellung ebenfalls Teil der vorliegenden Erfindung sind.

Die Verbindungen, worin R Isopropyl oder Aethyl ist, d. h. die Verbindungen 8-Isopropyl-1-oxaspiro [4,5] decan-2-on und 8-Aethyl-1-oxaspiro [4,5] decan-2-on, sind für den vorliegenden Zweck besonders bevorzugt. Beide riechen intensiv und sind von sehr guter Haftfestigkeit, wobei bezüglich dieser Eigenschaften das Aethyl-Analoge bevorzugt ist, weil es viel intensiver riecht. Die Gerüche dieser Verbindungen sind voneinander verschieden, doch sind sie komplementär, und die Verbindungen

können deshalb vorzugsweise als Gemisch verwendet werden. So kann beispielsweise eine geringe Menge des Aethyl-Analogen zu der Isopropyl-Verbindung gegeben werden, um in einer Riechstofformulierung die crèmige (sahnige) Note zu erhöhen, falls dies erwünscht ist.

Bei der Herstellung des Isopropyl-Analogen kann von handelsüblichem p-Isopropylcyclohexanol und von handelsüblicher Acrylsäure ausgegangen werden, wobei in diesem Fall ein Produkt anfällt, das ungefähr 35-55 % des cis-Isomeren und 45-65 % des trans-Isomeren (üblicherweise 40-50 % des cis-Isomeren und 50-60 % des trans-Isomeren) enthält. Der Geruch dieses Produktes kann als holzig, milchig-lactonartig, trocken (Pulver), intensiv und von guter Haftfestigkeit bezeichnet werden. Auf Grund dieser holzigen, milchig-lactonartigen Noten wird diese Verbindung vorzugsweise in einer Zahl sogenannter reicher Kompositionen, d. h. Kompositionen mit ausgesprochenem Körper, und zwar solchen von Edelholztyp, wie Sandelholztyp und Vetiver, als auch allgemein holzigem, trockenem, harzigen Typ, wie z. B. in Mooskompositionen eingesetzt. Ausserdem kann das 8-Isopropyl-1-oxaspiro [4.5] decan-2-on insbesondere auch in orientalischen Noten, in blumigen Bouquets und ähnlichem mit gutem Effekt eingesetzt werden.

Die Verbindung mit R = Aethyl fällt in einem ähnlichem Isomerenverhältnis an, wenn sie ausgehend von p-Aethylcyclohexanol hergestellt wird ; das Isomerenverhältnis ist üblicherweise 40-50 % cis-Isomeres und 50-60 % trans-Isomeres. Der Geruch dieses Produktes kann als intensiv, crèmig-lactonartig, leicht holzig bezeichnet werden. Das Aethyl-Analoge hat einen stärkeren crèmigen Charakter als das Isopropyl-Analoge ; es kann mit grossem Vorteil in solchen Riechstoffkompositionen eingesetzt werden, wo diese reiche crèmig-lactonartige Riechstoffqualität wünschenswert ist, also beispielsweise in blumigen Noten, wie in Gardenia, Jasmin und Tuberose, ferner in Edelholzkompositionen, wie in Sandelholzkompositionen. Auf Grund der Intensität und des Charakters seines Geruches kann das 8-Aethyl-1-oxaspiro [4.5] decan-2-on üblicherweise in geringen Mengen eingesetzt werden ; es erbringt bereits in solchen geringen Konzentrationen Fülle in einer ganzen Reihe von Riechstoffbasen.

In Formulierungen, in denen ein kräftiger, gehaltvoller, sahniger, holziger Komplex erwünscht ist, wird vorzugsweise ein Gemisch von 8-Aethyl-1-oxaspiro [4.5] decan-2- on und 8-Isopropyl-1-oxaspiro [4.5] decan-2-on eingesetzt. Wie oben gesagt, verträgt sich das Aethyl-Analoge gut mit dem Isopropyl-Analogen, insbesondere dort, wo crèmige Noten unterstrichen werden sollen. Im Falle dieses Gemisches muss das Aethylderivat üblicherweise nur in geringen Mengen im Isopropylderivat vorhanden sein, da die Geruchsintensität des ersteren die Geruchsintensität des letzteren um ein Vielfaches übersteigt. Vorzugsweise werden deshalb ungefähr 0,05 bis 1 % des Aethylderivats in solchen Gemischen eingesetzt, wobei ungefähr 0,1 bis 0,5 % besonders bevorzugt sind. Die Anwesenheit des Aethylderivates in diesen Konzentrationen liefert die gewünschte crèmige Note, so z. B. insbesondere in Formulierungen des Edelholztyps (z. B. Sandelholz), worin ein « reicher », crèmiger, holziger Komplex als ursächlich für die natürliche Sandelholznote angesehen wird. Selbstverständlich können auch höhere Mengen des Aethylderivats in der Formulierung vorhanden sein, insbesondere dann, wenn die crèmige Note noch mehr in den Vordergrund gerückt werden soll.

Auch das Methylderivat und das sek-Butylderivat stellen bevorzugte Verbindungen dar. Im Falle des Methylderivats, d. h. der Verbindung 8-Methyl-1-oxaspiro [4.5] decan-2-on, beträgt das Isomerenverhältnis im nach üblichen Methoden zugänglichen Produkt ungefähr 40 % cis und ungefähr 60 % trans. Dieses Produkt weist einen einzigartigen Geruch auf. Es hat zwar milchig-lactonartige Noten, die an das Aethylderivat erinnern, aber sein Geruch weist auch Richtung Coumarin, leicht cresylartig und andererseits ist die Verbindung weniger fruchtig als das Aethyl-Analoge. Das Methyl-Analoge ist um ein mehrfaches weniger intensiv als das 8-Aethyl-1-oxaspiro [4.5] decan-2-on. Auf Grund seines im Grunde crèmig, lactonartiges Charakters hat es sich als wertvoll für Riechstoffkompositionen erwiesen, in denen genau diese crèmig lactonartige Note erwünscht ist. Auf der andern Seite kann es insbesondere auch in solchen Formulierungen verwendet werden, in denen die Coumarinnote erwünscht ist.

Bei der Herstellung des neues 8-sek-Butyl-1-oxaspiro [4.5] decan-2-ons ausgehend aus p-sek-Butylcyclohexanol und Acrylsäure fällt ein Isomerengemisch mit ungefähr 30-45 % cis- und ungefähr 55-70 % trans-Isomeren an, wobei das üblicherweise erhaltene Gemisch 33-43 % cis- und 57-67 % trans-Isomeres enthält. Der Geruch dieser Gemische ist fruchtig, Richtung Pfirsich und Aprikose. Das sek-Butyl-Analoge wird deshalb demgemäss insbesondere dort eingesetzt, wo natürliche fruchtige Noten erwünscht sind. Es handelt sich dabei um Blumenbouquets, wie Weisse Rose, Jasmin, Narzisse, etc. ; in diesem Bouquet resultiert die Anwesenheit des Spirolactons in natürlichem Charakter. Zusätzlich zu den oben aufgeführten Eigenschaften hat sich das 8-sek-Butyl-1-oxaspiro [4.5] decan-2-on als wertvoll erwiesen in Riechstoffkompositionen, bei denen es darauf ankommt, dass vorhandene Noten entweder gut miteinander verbunden werden, und/oder dass gewisse Noten hervorgehoben werden sollen. Beispielsweise kann in Edelholznoten, z. B. vom Sandelholz- und Vetiver-Typ, das sek-Butyl-Analoge mit Vorteil verwendet werden, um die vorhandenen Noten weicher und/oder auch modifiziert erscheinen zu lassen. In kostbaren Formulierungen, wie solchen vom Vetiver-Typ, erhält man andererseits durch Zugabe des 8-sek-Butyl-Analogen fruchtige Noten, die sich äusserst gut mit den holzigen, moosigen und Zitrusnoten des Chypretyps verbinden und auch eine gewisse angenehme Modifizierung erzeugen.

Eine ganze Reihe dieser wünschenswerten Effekte in Riechstoffkompositionen wird in den unten stehenden Beispielen exemplifiziert ; es handelt sich dort insbesondere um Formulierungen vom Vetiver-, Sandelholz-, Moos-, Ambra- und Weisse Rosen-Typ.

In Sandelholz-, Vetiver- und Moos-Kompositionen wird sowohl eine bessere Verbindung der einzelnen Komponenten als auch mehr Kraft und Gehalt festgestellt. Zusätzlich bringt in diesen Kompositionen jedes der verwendeten Spirolactone der Formel I seine individuelle Note in die Formulierung ein. In den Sandelholz- und den Vetiver-Basen erzeugt das 8-Isopropyl-1-oxaspiro [4.5] decan-2-on, z. B. in Konzentrationen von 10 bzw. 20 %, sowohl eine Kräftigung als auch eine Verstärkung des Edelholzcharakters, begleitet von einer weichen sahnigen Note. In der moosartigen Formulierung tragen z. B. 15 % des Isopropylspirolactons zur Abrundung des dominierenden Holzcharakters bei. Das 8-Aethyl-1-oxaspiro [4.5] decan-2-on, z. B. 0,6 % und 1,2 %, bringt eine süsse crèmige Note im Falle der Sandelholz- und der Vetiver-Base : Die so behandelten Basen werden insbesondere als intensiver beschrieben, was speziell für den Vetiver-Typ zutrifft. Auch die Coumarinnote des Methylderivats eignet sich sehr gut für die Vetiverbase. Wenn man z. B. 4 % des Coumarins aus der Vetiverbase entfernt und an dessen Stelle 23 % des Methylspirolactons zugibt, wird nicht nur sofort eine kräftige gehaltvolle Lactonnote festgestellt, sondern es erscheint ein Grossteil der süssen Heunote des Coumarins wieder.

In Ambra- und Weisse-Rose-Kompositionen genügen geringe Mengen der Spirolactone I, um interessante Nuancen zu kreieren. In Ambra-Kompositionen führt der Gehalt an Spirolactonen I zu sahnigeren und ausbalancierteren Noten. Diesbezüglich muss insbesondere das Methylspirolacton erwähnt werden, welches bereits in einer Konzentration von nur 1 % Körper und Fülle zeitigt, währenddem die sahnige und holzige Note des Isopropyl-Analogen Weichheit und Vornehmheit ergibt. In dieser Base und in der Weissen Rosebase kann insbesondere demonstriert werden, wie geruchsstark das Aethylspirolacton ist, und welch geringe Menge bereits genügt, um wertvolle Effekte zu erzielen :

Eine Ambrabase wird schon bei Mengen von 0,05 % des Aethyl-Analogen kräftiger und gehaltvoller sowie auch sahniger, währenddem im Falle der Weissen Rose 0,03 % genügen, um mehr Natürlichkeit und auch eine gewisse fettige Note zu erzeugen.

Die Komponenten der Weissen Rose werden im allgemeinen als besser verbunden und natürlicher bezeichnet, wobei hier das Isopropyl-Analoge bei 0,5 % einen ähnlichen Effekt wie das Aethyl-Analoge zeitigt, d. h. es verstärkt den natürlichen blumigen Charakter, und die erhaltene Komposition erinnert in der Folge mehr an natürliches Rosenöl. Das 8-sek-Butyl-1-oxaspiro [4.5] decan-2-on, ebenfalls in einer Konzentration von 0,5 %, führt zu einem harmonischeren und fruchtigeren Charakter, wobei nun insbesondere eine natürliche Pfirsichnote festgestellt werden kann.

Die Verbindungen der Formel I können in Riechstoffkompositionen in Konzentrationen von ungefähr 0,01 bis 30 % eingesetzt werden. Im Falle des sehr intensiven Aethyl-Analogen ist der bevorzugte Bereich ungefähr 0,01 bis 5 %, für die restlichen Derivate ist der zweckmässige Bereich ungefähr 0,5 bis 30 %.

Der jeweils zweckmässigste Konzentrationsbereich in einer bestimmten Riechstoffbase ergibt sich natürlich insbesondere auch durch die Art der gewünschten Note und die Art des gewünschten Effekts. Aber auch der höhere Konzentrationsbereich, also derjenige von ungefähr 5-30 % soll nicht als limitierend verstanden werden, da der kreative Parfumeur in speziellen Fällen auch mit höheren Konzentrationen noch wertvolle Effekte erzielen kann.

Die Riechstoffkompositionen, die Verbindungen der Formel I enthalten, können beispielsweise als Riechstoffbasen für die Herstellung von Parfums und Toilettenwässer dienen, wobei in diesen Fällen die üblichen alkoholischen und wässrigen Verdünnungen angezeigt sind, mit andern Worten, ungefähr 15 bis 20 Gew.-% der Base wird im Falle der Parfumherstellung eingesetzt, und ungefähr 2-5 % der Base wird im Falle des Toilettenwassers eingesetzt.

Andererseits können die Riechstoffbasen aber auch benützt werden, um Produkte, wie Seifen, Detergentien, Kosmetika, etc. zu parfümieren. In diesen Fällen ist bekanntlich eine Konzentration von ungefähr 0,5 bis ungefähr 2 % der Riechstoffbase meistens genügend.

Die folgenden Beispiele exemplifizieren insbesondere bevorzugte Aspekte der vorliegenden Erfindung. Sie sollen deshalb als illustrativ und nicht als limitierend verstanden werden. Im Falle der Synthesebeispiele wurde die Analyse der Reaktionsprodukte unter Verwendung eines Perkin Elmer 900 Gaschromatographen mittels einer 1/16 Inch × 500 Fuss, 2 % Carbowax 20M-Kolonne oder einer 1/8 × 20 Fuss 2 % OV-101-Kolonne durchgeführt [1 Inch = 2,54 cm, 1 Fuss = 30,48 cm]. Die Geruchsangaben der einzelnen Isomeren beziehen sich auf das Isomere, das jeweils aus dem Gaschromatographen eluiert werden konnte.

### Beispiel 1

Herstellung der Oxaspiro [4.5] decan-2-one (I)

I-a. 8-Isopropyl-1-oxaspiro [4.5] decan-2-on

Zu 1,247 kg 4-Isopropylcyclohexanol wurde bei 143-150 °C innert 6 Stunden ein Gemisch von 71,1 g (1 Mol) Acrylsäure, 142 g (1 Mol) 4-Isopropylcyclohexanol und 14,6 g (0,1 Mol) di-tert-Butylperoxid gegeben. Nach einer zusätzlichen Reaktionszeit von 1 Stunde bei 143-150 °C wurde das Reaktionsgemisch unter vermindertem Druck destilliert, wobei 142,1 g Produkt resultierten. Siedepunkt 125-141 °C/1,0 mmHg. Geruch : holzig, milchig-lactonartig, trocken. Analyse : Reinheit 99 % (gaschromatographisch ; 44 % cis (Geruch : Nonalacton, Sellerielacton), 56 % trans (Geruch : stark holzig, zimtartig,

urinös-animalisch).

Analog wurden erhalten :

I-b. 8-Methyl-1-oxaspiro [4.5] decan-2-on

Siedepunkt 97-100 °C/0,5 mmHg, Geruch : stark, sahnig, lacton-artig, Coumarin, leicht cresyl-artig. Analyse : > 99 % rein (Gaschromatographie). 40 % cis (Geruch : reich, holzig, lacton-artig). 60 % trans (Geruch : urinös-animalisch), milchig-lacton-artig, leicht cresylartig).

I-c. 8-Aethyl-1-oxaspiro [4.5] decan-2-on

Siedepunkt 121-130 °C/0,5 mmHg. Geruch : intensiv, sahnig, lacton-artig, leicht holzig. Analyse : 99 % rein ; 42 % cis (Geruch : sahnig, pfirsich-lacton-artig). 58 % trans (Geruch : leicht nach Zimtaldehyd, sahnig-lactonartig).

I-d. 8-sek-Butyl-1-oxaspiro [4.5] decan-2-on

Siedepunkt : 164-180 °C/0,5 mmHg. Geruch : nach Pfirsich, Aprikose, lacton-artig. Analyse : Reinheit 99 % ; 37 % cis (Geruch : nach Nonalacton, Sellerielacton). 63 % trans (Geruch : leicht lederig, urinös-animalisch).

I-e. 8-tert-Butyl-1-oxaspiro [4.5] decan-2-on

Siedepunkt 140-142 °C/0,5 mmHg. Geruch : holzig, Ambra, Analyse : 99 % Reinheit ; 32 % cis (Geruch : lacton-artig, Pfirsich-artig, nach Nonalacton). 68 % trans (Geruch : urinös-animalisch, holziglactonartig).

## Beispiel 2

Verbindungen der Formel I in Riechstoffkompositionen

A. Vetiver-Base

| Komponenten | Gewichtsteile |
|---|---|
| Vetiveröl Bourbon | 290 |
| Santalol | 200 |
| Hydroxycitronellal | 90 |
| Zimtalkohol | 50 |
| Heliotropin | 60 |
| Coumarin | 30 |
| Keton-moschus (4-t-Butyl-3,5-dinitro-2,6-dimethylacetophenon) | 50 |
| Ambrette-moschus (6-t-Butyl-3-methyl-2,4-dinitroanisol) | 30 |
| | 800 |

Die so formulierte Base hinterliess keinen harmonischen Eindruck. Insbesondere fehlte das verbindende Element zwischen Vetiveröl und den anderen Ingredienzien : Die Ingredienzien schienen nicht gut miteinander verbunden und in der Komposition wurden sogenannte chemische Noten festgestellt.

Die Zugabe von 9,96 Teilen (1,2 %) 8-Aethyl-1-oxaspiro [4.5] decan-2-on verband die einzelnen Noten und resultierte in Süsse and Sahnigkeit. Die so erhaltene Vetiverbase erschien intensiver und natürlicher.

Die Zugabe von 200 Teilen (20 %) 8-Isopropyl-1-oxaspiro [4.5] decan-2-on verband die « misstönenden » Noten ebenfalls und der Edelholzcharakter wurde verstärkt. Der allgemeine Eindruck der erhaltenen Base war : Weicher und sahniger, natürlicheres Vetiver. Wenn anstelle des reinen Isopropylderivats 200 Teile eines Gemisches aus 99,9 bis 99,5 % 8-Isopropyl-1-oxaspiro [4.5] decan-2-on und 0,1 % bis 0,5 % 8-Aethyl-1-oxaspiro [4.5] decan-2-on verwendet wurde, wirkte die resultierende Base ebenfalls sahniger und intensiver sowie auch natürlicher.

Nun wurde die obige Base noch ohne die 30 Teile Coumarin formuliert. Die Formulierung wurde als « dünner », holziger und ohne den Heu-Effekt des Coumarins charakterisiert. Zugabe von 230 Teilen (23 %) von 8-Methyl-1-oxaspiro [4.5] decan-2-on zeitigte nun einen äussert wünschenswerten Effekt, indem die süsse Coumarin-ähnliche Note erzeugt wurde, aber mehr Lactoncharakter festgestellt wurde ; die Vetiverbase erschien eindeutig harmonischer.

B. Sandelholzbase

| Komponenten | Gewichtsteile |
|---|---|
| Sandalore® Givaudan [3-Methyl-5-(2,2,3-trimethylcyclopent-3-en-1-yl)-pentan-2-ol] | 300 |
| Ambrette-moschus (6-t-Butyl-3-methyl-2,4-dinitroanisol) | 50 |
| Amyrisöl | 20 |
| Hydroxycitronellal | 30 |
| Methyljonon | 100 |
| Benzyl-benzoat | 400 |
| | 900 |

Diese Sandelholzbase erschien etwas « dünn », d. h. es fehlte ihr Körper und Fülle.

Zugabe von 5 Teilen (0,6 %) von 8-Aethyl-1-oxaspiro [4.5] decan-2-on erhöhte nun die Sahnigkeit der Riechstoffkomposition, zeitigte Fülle und eine viel natürlichere Sandelholzkörper-Kopfnote.

Zugabe von 100 Teilen (10 %) 8-Isopropyl-1-oxaspiro [4.5] decan-2-on erhöhte den holzigen Charakter und resultierte in einer sahnigen Note als auch in Fülle, wodurch die Riechstoffbase stark verbessert erschien. Im Falle der Verwendung von 100 Teilen 8-Isopropyl-1-oxaspiro [4.5] decan-2-on mit einem Gehalt an ungefähr 0,1 bis 0,5 % 8-Aethyl-1-oxaspiro [4.5] decan-2-on erschien die sahnige Note noch prononcierter und die resultierende Sandelholzbase wurde als noch natürlicher taxiert.

C. Moosbase

| Komponenten | Gewichtsteile |
|---|---|
| Citronenöl-Terpene | 70 |
| Limetteöl (destilliert) | 50 |
| Orangenöl (Californ.) | 30 |
| Amyl-salicylat raffiniert | 20 |
| Benzyl-salicylat | 30 |
| Coumarin | 50 |
| Heliotropin | 80 |
| Folione ®Givaudan (Methyl-2-octynoat) | 10 |
| 4-Acetyl-6-t-butyl-1,1-dimethyl-indan | 40 |
| Eichenmoos (lösliches Harz) | 50 |
| Lilial ®Givaudan (p-t-Butyl-α-methylhydrocinnamaldehyd) | 100 |
| Phenyläthyl-alkohol | 100 |
| α-Terpineol | 60 |
| Hydroxycitronellal | 40 |
| Diäthyl-phthalat | 120 |
| | 850 |

In der obigen Base dominierte der holzige Charakter, dessen Komponenten im übrigen nicht gut miteinander verbunden waren. Die Zugabe von 150 Teilen (15 %) von 8-Isopropyl-1-oxaspiro [4.5] decan-2-on balancierten nun den Mooscharakter aus, indem die dominierende Holznote gut in die Base integriert wurde und nun viel harmonischer wirkte.

D. Amber-Base

| Komponenten | Gewichtsteile |
|---|---|
| Labdanum-Harz (löslich) | 376,3 |
| Patchouli-öl Moyenne (Gemisch verschiedener Provenienz) | 19,8 |
| Benzoe-harz löslich | 99,0 |
| Vetiver-öl (Bourbon) | 49,5 |
| Sandelholz-öl (ostindisch) | 99,0 |
| Mousse de Chêne Absolue (50 % in Aethanol) | 19,8 |
| Ambrette-moschus (6-t-Butyl-3-methyl-2,4-dinitroanisol) | |
| Keton-Moschus (4-t-Butyl-3,5-dinitro-2,4-dimethylacetophenon) | 49,5 |
| Vanillin | 49,5 |
| Bergamotte-öl nicht-sensibilisierend (Furocoumarin-frei) | 69,3 |
| Orangenöl (Californ.) (kalt gepresst) | 49,5 |
| Rosenbase [hauptsächlich Phenyläthylalkohol, Citronellol, Phenylacetat] | 49,5 |
| Castoreum Absolue (10 % in Aethanol) | 9,9 |
| | 990,1 |

In der obigen Amber-Base wurden citrusartige, animalische und süsse Noten festgestellt, die aber

nicht gut verbunden erschienen. Zugabe von 9,9 Teilen (1 %) von 8-Methyl-1-oxaspiro [4.5] decan-2-on oder 8-Isopropyl-1-oxaspiro [4.5] decan-2-on verband diese misstönenden Noten in der Komposition. Das Methylspirolacton verstärkte die Base, d. h. es resultierte Fülle und Körper, sahnigerer und süsserer Charakter. Das Isopropylspirolacton zeitigte einen kräftigeren, gehaltvolleren, sahnigen, leicht holzigen Charakter, der zur Erzeugung der weichen Amber-Note als unabdingbar erschien.

Auch die Zugabe von 0,49 Teilen (0,05 %) 8-Aethyl-1-oxaspiro [4.5] decan-2-on ergab in der Amber-Base einen sahnigen, leicht holzigen Charakter, diesmal aber mit einem stärkeren Lactoneffekt. Auch die Labdanumnoten waren nun besser feststellbar.

E. Weisse Rose

| Komponenten | Gewichtsteile |
|---|---|
| Palmarosa-öl | 400 |
| « Ginger Grass-öl » (ex Cymbopogon Martini) | 100 |
| Geranium-öl algerisch | 50 |
| Citronellol | 150 |
| Benzyl-acetat | 100 |
| Phenyläthyl-acetat | 60 |
| Phenyläthyl-alkohol | 40 |
| Aldehyd-Gemisch (gleiche Gewichsteile von n-Octanal, n-Nonanal und n-Decanal) | 5 |
| Methyljonon | 20 |
| α-Jonon | 60 |
| Nonyl-alkohol | 10 |
| | 995 |

Die so formulierte Weisse-Rosen-Base enthielt nicht miteinander verbundene fruchtige Noten. Wenn die Base jedoch unter Verwendung von 0,25 Teilen (0,03 %) 8-Aethyl-1-oxaspiro [4.5] decan-2-on formuliert wurde, bzw. mit 5 Teilen (0,5 %) 8-Isopropyl-1-oxaspiro [4.5] decan-2-on, oder mit 8-sek-Butyl-1-oxaspiro [4.5] decan-2-on, wurden diese vorher misstönenden Noten als gut miteinander verbunden bezeichnet. Das 8-Aethylspirolacton oder das Isopropylspirolacton erhöhten auch den natürlichen Charakter der Rosenbase, indem der gewünschte fettige bzw. sahnige Charakter erzeugt wurde. Das sek.-Butylspirolacton erhöhte den fruchtigen Charakter, indem der Komposition eine natürliche Pfirsichnote verliehen wurde. Sämtliche auf diese Weise erhaltenen Riechstoffbasen wurden als eindeutig gelungener bezeichnet.

**Patentansprüche**

1. Riechstoffkomposition, gekennzeichnet durch einen Gehalt an mindestens einer Verbindung der Formel

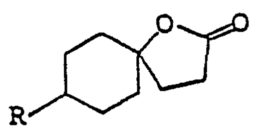

(I)

worin R Methyl, Aethyl, Isopropyl, sek. Butyl oder tert. Butyl darstellt.

2. Riechstoffkomposition gemäss Anspruch 1, gekennzeichnet durch einen Gehalt an 8-Methyl-1-oxaspiro [4.5] decan-2-on.

3. Komposition gemäss Anspruch 2, dadurch gekennzeichnet, dass das 8-Methyl-1-oxaspiro [4.5] decan-2-on zu 35-45 % aus dem cis-Isomeren und zu 55-65 % aus dem trans-Isomeren besteht.

4. Riechstoffkomposition gemäss Anspruch 1, gekennzeichnet durch einen Gehalt an 8-Aethyl-1-oxaspiro [4.5] decan-2-on.

5. Riechstoffkomposition gemäss Anspruch 4, dadurch gekennzeichnet, dass das 8-Aethyl-1-oxaspiro [4.5] decan-2-on zu 40-50 % aus dem cis-Isomeren und zu 60-50 % aus dem trans-Isomeren besteht.

6. Riechstoffkomposition gemäss Anspruch 1, gekennzeichnet durch einen Gehalt an 8-Isopropyl-1-oxaspiro [4.5] decan-2-on.

7. Riechstoffkomposition gemäss Anspruch 6, dadurch gekennzeichnet, dass das 8-Isopropyl-1-oxaspiro [4.5] decan-2-on zu 35-55 %, vorzugsweise zu 40-50 %, aus dem cis-Isomeren und zu 45-65 %, vorzugsweise zu 50-60 %, aus dem trans-Isomeren besteht.

8. Riechstoffkomposition gemäss Anspruch 1, gekennzeichnet durch einen Gehalt an 8-sek. Butyl-1-oxaspiro [4.5] decan-2-on.

9. Riechstoffkomposition gemäss Anspruch 8, dadurch gekennzeichnet, dass das 8-sek.Butyl-1-oxaspiro [4.5] decan-2-on zu 30-45 %, vorzugsweise zu 33-43 %, aus dem cis-Isomeren und zu 55-70 %,

vorzugsweise zu 55-67 %, aus dem trans-Isomeren besteht.

10. Riechstoffkomposition gemäss Anspruch 1, gekennzeichnet durch ein Gemisch von 8-Isopropyl-1-oxaspiro [4.5] decan-2-on und 8-Aethyl-1-oxaspiro [4.5] decan-2-on.

11. Riechstoffkomposition gemäss Anspruch 10, dadurch gekennzeichnet, dass das 8-Isopropyl-1-oxaspiro [4,5] decan-2-on [40-50 % cis Isomeres und 50-60 % trans-Isomeres] und das 8-Aethyl-1-oxaspiro [4.5] decan-2-on [40-50 % cis-Isomeres und 50-60 % trans-Isomeres] zueinander im Gewichtsverhältnis 99 : 1 bis 99,95 : 0,05 stehen.

12. Verwendung einer Verbindung der Formel

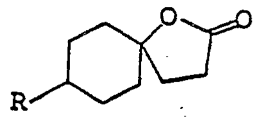

(I)

worin R Methyl, Aethyl, Isopropyl, sek.Butyl oder tert.Butyl darstellt, als Riechstoff.

13. Verwendung von 8-Methyl-1-oxaspiro [4.5] decan-2-on als Riechstoff.

14. Verwendung von 8-Aethyl-1-oxaspiro [4.5] decan-2-on als Riechstoff.

15. Verwendung von 8-Isopropyl-1-oxaspiro [4.5] decan-2-on als Riechstoff.

16. Verwendung von 8-sek. Butyl-1-oxaspiro [4.5] decan-2-on als Riechstoff.

17. Verbindungen der Formel

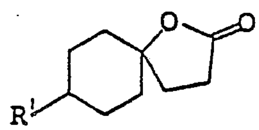

(I')

worin R' Methyl, Aethyl oder sek.Butyl bedeutet.

18. 8-Methyl-1-oxaspiro [4.5] decan-2-on.

19. 8-Aethyl-1-oxaspiro [4.5] decan-2-on.

20. 8-sek.Butyl-1-oxaspiro [4.5] decan-2-on.

21. Verfahren zur Herstellung von Verbindungen der Formel

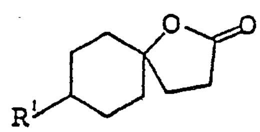

(I')

worin R' Methyl, Aethyl oder sek.Butyl bedeutet, dadurch gekennzeichnet, dass man Acrylsäure oder einen Ester der Acrylsäure mit einer Verbindung der Formel

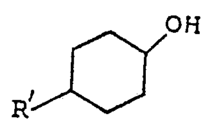

(II')

worin R' obige Bedeutung besitzt, umsetzt.

**Claims**

1. An odorant composition, characterized by a content of at least one compound of the formula

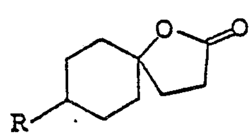

(I)

wherein R represents methyl, ethyl, isopropyl, sec.butyl or tert.butyl.

2. An odorant composition in accordance with claim 1, characterized by a content of 8-methyl-1-oxaspiro [4.5] decan-2-one.

3. A composition in accordance with claim 2, characterized in that the 8-methyl-1-oxaspiro [4.5] decan-2-one consists to 35-45 % of the cis isomer and to 55-65 % of the trans isomer.

4. An odorant composition in accordance with claim 1, characterized by a content of 8-ethyl-1-oxaspiro [4.5] decan-2-one.

5. An odorant composition in accordance with claim 4, characterized in that the 8-ethyl-1-oxaspiro

[4.5] decan-2-one consists to 40-50 % of the cis isomer and to 60-50 % of the trans isomer.

6. An odorant composition in accordance with claim 1, characterized by a content of 8-isopropyl-1-oxaspiro [4.5] decan-2-one.

7. An odorant composition in accordance with claim 6, characterized in that the 8-isopropyl-1-oxaspiro [4.5] decan-2-one consists to 35-55 %, preferably to 40-50 %, of the cis isomer and to 45-65 %, preferably to 50-60 %, of the trans isomer.

8. An odorant composition in accordance with claim 1, characterized by a content of 8-sec.butyl-1-oxaspiro [4.5]-decan-2-one.

9. An odorant composition in accordance with claim 8, characterized in that the 8-sec.butyl-1-oxaspiro [4.5]-decan-2-one consists to 30-45 %, preferably to 33-43 %, of the cis isomer and to 55-70 %, preferably to 55-67 %, of the trans isomer.

10. An odorant composition in accordance with claim 1, characterized by a mixture of 8-isopropyl-1-oxaspiro [4.5]-decan-2-one and 8-ethyl-1-oxaspiro [4.5] decan-2-one.

11. An odorant composition in accordance with claim 10, characterized in that the 8-isopropyl-1-oxaspiro [4.5]-decan-2-one [40-50 % of cis isomer and 50-60 % of trans isomer] and the 8-ethyl-1-oxaspiro [4.5] decan-2-one [40-50 % of cis isomer and 50-60 % of trans isomer] are present to each other in the weight ratio 99 : 1 to 99.95 : 0.05.

12. The use of a compound of the formula

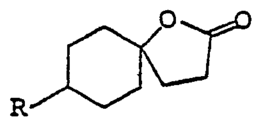

(I)

wherein R represents methyl, ethyl, isopropyl, sec.butyl or tert.butyl, as an odorant.

13. The use of 8-methyl-1-oxaspiro [4.5] decan-2-one as an odorant.

14. The use of 8-ethyl-1-oxaspiro [4.5] decan-2-one as an odorant.

15. The use of 8-isopropyl-1-oxaspiro [4.5] decan-2-one as an odorant.

16. The use of 8-sec.butyl-1-oxaspiro [4.5] decan-2-one as an odorant.

17. Compounds of the formula

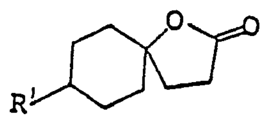

(I')

wherein R' signifies methyl, ethyl or sec.butyl.

18. 8-Methyl-1-oxaspiro [4.5] decan-2-one.

19. 8-Ethyl-1-oxaspiro [4.5] decan-2-one.

20. 8-sec.Butyl-1-oxaspiro [4.5] decan-2-one.

21. A process for the manufacture of compounds of the formula

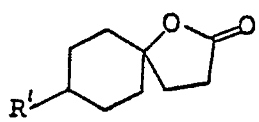

(I')

wherein R' signifies methyl, ethyl or sec.butyl, characterized by reacting acrylic acid or an ester of acrylic acid with a compound of the formula

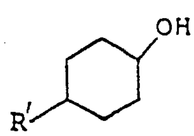

(II')

wherein R' has the above significance.

**Revendications**

1. Composition odorante caractérisée en ce qu'elle contient au moins un composé de formule

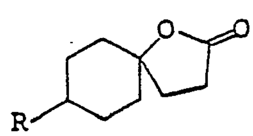

(I)

dans laquelle R est le méthyle, éthyle, isopropyle, butyle séc- ou tertio-butyle.

2. Composition odorante selon la revendication 1, caractérisée en ce qu'elle contient de la 8-méthyl-1-oxaspiro [4,5] décanone-2.

3. Composition selon la revendication 2, caractérisée en ce que la 8-méthyl-1-oxaspiro [4,5] décanone-2 est constituée de 35-45 % de l'isomère-cis et de 55-65 % de l'isomère-trans.

4. Composition odorante selon la revendication 1, caractérisée en ce qu'elle contient de la 8-éthyl-1-oxaspiro [4,5] décanone-2.

5. Composition odorante selon la revendication 4, caractérisée en ce que la 8-éthyl-1-oxaspiro [4,5] décanone-2 est constituée de 40-50 % de l'isomère-cis et de 60-50 % de l'isomère-trans.

6. Composition odorante selon la revendication 1, caractérisée en ce qu'elle contient de la 8-isopropyl-1-oxaspiro [4,5] décanone-2.

7. Composition odorante selon la revendication 6, caractérisée en ce que la 8-isopropyl-1-oxaspiro [4,5] décanone-2 est constituée de 35-55 %, de préférence de 40-50 % de l'isomère cis et de 45-65 %, de préférence de 50-60 % de l'isomère trans.

8. Composition odorante selon la revendication 1, caractérisée en ce qu'elle contient de la 8-(butyl sec.)-1-oxaspiro [4,5] décanone-2.

9. Composition odorante selon la revendication 8, caractérisée en ce que la 8-(butyl sec.)-1-oxaspiro [4,5] décanone-2 est constituée de 30-45 %, de préférence 33-43 % de l'isomère cis et de 55-70 %, de préférence 55-67 % de l'isomère trans.

10. Composition odorante selon la revendication 1, caractérisée par un mélange de 8-isopropyl-1-oxaspiro [4,5] décanone-2 et de 8-éthyl-1-oxaspiro [4,5] décanone-2.

11. Composition odorante selon la revendication 10, caractérisée en ce que la 8-isopropyl-1-oxaspiro [4,5] décanone-2 [40-50 % d'isomère cis et 50-60 % d'isomère trans] et la 8-éthyl-1-oxaspiro [4,5] décanone-2 [40-50 % d'isomère cis et 50-60 % d'isomère trans] sont dans un rapport pondéral mutuel de 99 : 1 à 99,95 : 0,05.

12. Utilisation comme parfum d'un composé de formule

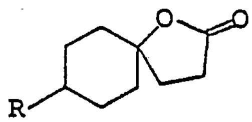

(I)

dans laquelle R est le méthyle, éthyle, isopropyle, butyle sec. ou tertio-butyle.

13. Utilisation comme parfum de 8-méthyl-1-oxaspiro [4,5] décanone-2.

14. Utilisation comme parfum de 8-éthyl-1-oxaspiro [4,5] décanone-2.

15. Utilisation comme parfum de 8-isopropyl-1-oxaspiro [4,5] décanone-2.

16. Utilisation comme parfum de 8-(butyl sec.)-1-oxaspiro [4,5] décanone-2.

17. Composés de formule

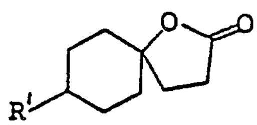

(I')

dans laquelle R' est le méthyle, éthyle ou butyle secondaire.

18. 8-méthyl-1-oxaspiro [4,5] décanone-2.

19. 8-éthyl-1-oxaspiro [4,5] décanone-2.

20. 8-butyle sec.-1-oxaspiro [4,5] décanone-2.

21. Procédé de préparation de composés de formule

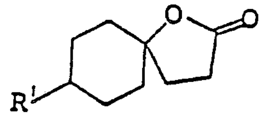

(I')

dans laquelle R' est le méthyle, éthyle ou butyle sec. caractérisé en ce qu'on fait réagir de l'acide acrylique ou un ester de l'acide acrylique avec un composé de formule

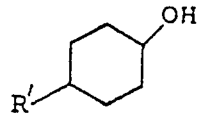

(II')

dans laquelle R' a la signification ci-dessus.